# EUROPEAN PATENT SPECIFICATION

(11) **EP 3 315 946 B1**
(45) Date of publication and mention of the grant of the patent: **08.07.2020**
(21) Application number: 16813640.6
(22) Date of filing: 27.05.2016
(51) Int. Cl.: G01N 21/33, G01N 21/78, G01N 33/15, G01N 13/00

(54) **METHOD FOR DETECTING DISSOLUTION RATE OF PREPARATION CONTAINING COLLOIDAL BISMUTH PECTIN**
VERFAHREN ZUR ERKENNUNG DER AUFLÖSUNGSGESCHWINDIGKEIT EINES PRÄPARATS MIT KOLLOIDALEM WISMUTPEKTIN
PROCÉDÉ DE DÉTECTION DE TAUX DE DISSOLUTION DE PRÉPARATION CONTENANT DE LA PECTINE DE BISMUTH COLLOÏDALE

(30) Priority: 23.06.2015 CN 201510348307
(43) Date of publication of application: 02.05.2018
(73) Proprietor: Shanxi Zhendong Ante Biopharmaceutical Co. Ltd., Jinzhong, Shanxi 030600 (CN)
(72) Inventor: LI, Anping, Jinzhong, Shanxi 030600 (CN); CUI, Feng, Jinzhong, Shanxi 030600 (CN); ZHU, Ping, Jinzhong, Shanxi 030600 (CN); QIN, Zhengguo, Jinzhong, Shanxi 030600 (CN); ZHENG, Tai, Jinzhong, Shanxi 030600 (CN); WU, Yuexia, Jinzhong, Shanxi 030600 (CN)
(74) Representative: Klunker IP Patentanwälte PartG mbB
(86) International application number: PCT/CN2016/083740
(87) International publication number: WO 2016/206524

(56) References cited:
- CN-A- 101 008 610
- CN-A- 104 147 041
- CN-A- 104 897 668
- GB-B- 2 446 166
- US-B1- 6 762 841
- YANRU YUN ET AL: "Determination of bismuth in pharmaceutical products using phosphoric acid as molecular probe by resonance light scattering : Determination of bismuth by resonance light scattering", LUMINESCENCE: THE JOURNAL OF BIOLOGICAL AND CHEMICAL LUMINESCENCE, vol. 27, no. 5, 23 September 2011 (2011-09-23), pages 352-356, XP055541919, GB ISSN: 1522-7235, DOI: 10.1002/bio.1357
- ZHANG, HUA ET AL.: 'Improvement of determination method of dissolution of ranitidine bismuth potassium citrate tablet' GUIDE OF CHINESE MEDICINE vol. 6, no. 19, 31 October 2008, pages 87 - 89, XP009507928

## Description

### BACKGROUND OF THE PRESENT INVENTION

### Field of Invention

The present invention relates to a method for detecting a dissolution rate, and more particularly to a method for detecting a dissolution rate of a preparation containing colloidal bismuth pectin.

### Description of Related Arts

YUNRU YUN ET AL discloses "Determination of bismuth in pharmaceutical products using phosphoric acid as molecular probe by resonance light scattering", see LUMINESCENCE: THE JOURNAL OF BIOLOGICAL AND CHEMICAL LUMINESCENCE, vol. 27, no. 5, 1 September 2012, pages 352-356.

EP 3 181 140 A1 discloses a dispersion preparation containing colloidal bismuth pectin. Herein 1g is adopted as a unit for the preparation. Each unit of the preparation includes: 44.0 to 900.0 mg of colloidal bismuth pectin, 1.0-500 mg of a penetration enhancer, 2.0-312.5mg of acid-source pore forming agent, 2.0-250.0mg of alkali-source pore forming agent and 1.0-400.0mg of a disintegrating agent.

The colloidal bismuth pectin is a compound of uncertain constitution of the pectin and the bismuth Bi, which is yellow powder; wherein the bismuth pectin content (takes bismuth for calculation) is 14.0%-16.0%; pH is 8.5-10.5; the sedimentation rate is 1-0.97. The colloidal bismuth pectin is insoluble in ethanol, acetone, ether and other organic solvents, which is able to disperse uniformly and form stable colloidal system in the water.

The colloidal bismuth pectin substitutes small molecule acid groups with biological macromolecules pectin. Compared with other bismuth preparations such as bismuth subgallate, bismuth subnitrate, bismuth subsalicylate and bismuth potassium cirtrate, the colloidal bismuth pectin has strong colloidal characters, high viscosity and low absorption by human body. The colloidal pectin bismuth has high affinity for ulcerated mucosa, which forms chelation with the ulcer mucoprotein by bismuth to cover on the gastric mucosa. The epithelial tissues are stimulated to discharge mucus and the pepsin activity is inhibited to protect the gastric mucosa. The bismuth is able to kill the *Helicobacter pylor*i. Compared to the conventional medicine, the colloidal bismuth pectin has strong protection for mucosa, which widely applied in the treatment of intestinal tract disease including peptic ulcer disease, chronic gastritis and etc.

The colloidal bismuth pectin and the capsule preparation is an original ground medicine manufactured by the Taiyuan Red Star Pharmacy Plant which is the predecessor of Shanxi Zhendong Ante Biopharmaceutical Co., Ltd. The medicine gets the new drug certification and the drug production license from the Ministry of Health of the People's Republic of China in 1992 and now is listed in the Pharmacopoeia of the People's Republic of China, 2nd volume of the 2010 version. There is no dissolution detection event for the colloidal bismuth pectin preparations.

Quality of oral solid preparation is influenced by formulation design, preparation process, storage conditions and other factors, wherein dissolution and absorption processes determine its effectiveness. Dissolution and disintegration time limit are important indicators of a solid preparation control, but relying only on disintegration time limit test as a measure standard is clearly not perfect. Disintegration time limit is only the initial stage of drug dissolution, and subsequent dispersion and dissolution processes will not be reflected by disintegrating time limit test. However, dissolution test covers disintegration, dispersion and dissolution processes, so it is more important to study dissolution of solid preparations.

Colloidal bismuth pectin is a complex consists of biological macromolecules pectin and metal form bismuth, which is greatly different from generic drugs and is not easy to be absorbed by human body. However, a dissolution amount directly affects protection effect of the colloidal bismuth pectin on gastric mucosa and killing Helicobacter pylori. In addition, detection of the dissolution of colloidal bismuth pectin preparations can more objectively reflect the relationship and impacts between drug and excipients, which reflects impacts of formation process variations and storage conditions on pharmaceutical quality to ensure homogeneity, effectiveness and stability, and is of great significance.

However, the colloidal bismuth pectin formed by chelation of pectin and metal bismuth has very stable properties, colloid viscosity is high, and colloidal dispersion system is formed after dissolution in dissolution medium. Therefore, if dissolution is determined fully in accordance with "Pharmacopoeia of the People's Republic of China, 2010 Edition" combined with dissolution detection in Appendix XC, not only titration liquid is hardly consumed, but also the formed colloidal dispersion system has a great impact on judgment of complexometric titration end point, resulting in poor repeatability and low accuracy of the detection method. Furthermore, due to a size of a microporous membrane for filtering dissolution liquid is generally 200nm-1µm, recovery rate during dissolution detection is low. As a result, bismuth pectin dissolution rate of the preparation containing colloidal bismuth pectin can not be accurately measured, and the methods need to be improved.

### SUMMARY OF THE PRESENT INVENTION

An object of the present invention is to provide a method for detecting a dissolution rate of a preparation containing colloidal bismuth pectin, so as to detect a colloidal bismuth pectin dissolution rate of the preparation containing the colloidal bismuth pectin, in such a manner that a quality of the preparation containing the colloidal bismuth pectin is better controlled.

Accordingly, in order to accomplish the above objects, the present invention provides a method for detecting a dissolution rate of a preparation containing colloidal bismuth pectin, comprising steps of: according to "Pharmacopoeia of the People's Republic of China, 2010 Edition, Volume 2, Appendix XC", dispersing the preparation containing the colloidal bismuth pectin into dissolution medium, dissolving for 3-120min with a centrifuging rate of 30-150r/min, and directly obtaining dissolution liquid; adding a protonic acid dissociation agent into the dissolution liquid until a hydrogen ion concentration reaches 0.8-1.2mol/L; centrifuging after completely dissociation; separating a supernatant; coloring the supernatant by adding a chromogen solution of citric acid or ascorbic acid and potassium iodide to obtain a test solution; testing an absorbance of the test solution at a wavelength of 380-470nm; comparing the absorbance of the test solution with an absorbance of a reference solution with a known bismuth concentration under same conditions; calculating a dissolution amount, which is accounted by bismuth, of the colloidal bismuth pectin; wherein the dissolution medium is water, a buffer solution with a pH of 1.0-10.0, or an acid solution.

Furthermore, according to the present invention, the buffer solution is an acetate buffer solution, a phthalate buffer solution, a phosphate buffer solution or a borate buffer solution, with a pH of 1.0-10.0; and the acid solution is a 0.005-0.1mol/L hydrochloric acid solution.

According to the present invention, the dissolution detection method is a first method (Basket Apparatus), a second method (Paddle Apparatus) or a third method (Cup Apparatus) in the "Pharmacopoeia of the People's Republic of China, 2010 Edition, Volume 2, Appendix XC".

The protonic acid dissociation agent is nitric acid, hydrochloric acid or sulfuric acid. Preferably, the protonic acid dissociation agent is the nitric acid.

The dissolution liquid dissociated with the protonic acid is centrifuged for 5-15min at 7000-10000r/min. The dissociated polymeric pectin in the dissolution liquid is fully settled to form a bismuth test solution without disturbance, which fulfils test requirements of a spectrophotometry method.

According to the method of the present invention, the chromogen solution is a water solution or a 0.2-2mol/L nitric acid solution of potassium iodide, in which citric acid or ascorbic acid is added.

Furthermore, the chromogen solution comprises the citric acid or the ascorbic acid of 0.5wt%-10wt%, and the potassium iodide of 2.5wt%-25wt%.

Preferably, the chromogen solution comprises the citric acid or the ascorbic acid of 2.5wt%, and the potassium iodide of 12.5wt%.

According to the method of the present invention, the reference solution of the bismuth with a suitable concentration is prepared by dissolving the bismuth in the nitric acid before being diluted with water and adding the chromogen solution.

Specifically, the bismuth content per 1ml of the test solution or 1ml of the reference solution of the bismuth is 0.1-50µg. Preferably, the bismuth content per 1ml of the test solution or 1ml of the reference solution of the bismuth is 2-20µg. More preferably, the bismuth content per 1ml of the test solution or 1ml of the reference solution of the bismuth is 5-12µg.

A single-wavelength method is adopted for measurement of the bismuth content. Alternatively, a double-wavelength method may also be adopted to avoid disturbance.

Yellow bismuth potassium iodide generated by the bismuth and the potassium iodide has characteristic absorption spectroscopy at 399±2nm(crest), 433±2nm(trough), 463±2nm(crest). When the single-wavelength method is adopted for measurement; detection wavelengths are any wavelength from 399nm, 433nm and 463nm, preferably 463nm.

When a double-wavelength method is adopted for measurement; detection wavelengths are any two wavelengths from 399nm, 433nm and 463nm; wherein the content is calculated with absorbance difference. Preferably, a combination of 433nm and 463nm is chosen.

The method for detecting a dissolution rate of a preparation containing colloidal bismuth pectin is suitable for a single preparation or a compound preparation containing the colloidal bismuth pectin, comprising tablets, dispersible tablets, enteric-coated tablets, colon-enteric-coated tablets, capsules, soft capsules, enteric-coated capsules, colon-enteric-coated capsules, granules, dripping pills, microcapsules and dry suspension.

Colloidal bismuth pectin is a complex consists of biological macromolecules pectin and bismuth, a colloidal dispersion system is formed after dissolution in dissolution medium, and there is a huge difference between the colloidal bismuth pectin and basic bismuth gallate, basic bismuth nitrate, bismuth subsalicylate, bismuth potassium citrate as well as other small molecules. Therefore, after sampling dissolution liquid, the present invention directly adds proton acid for disaggregation treatment without filtering, in such a manner that metal form bismuth is separated from pectin. Then a high-speed centrifugation method is used, in such a manner that the pectin and excipients precipitate and are fully separated from bismuth.

Furthermore, the present invention takes the advantage of the characteristic reaction between bismuth and potassium iodide in acid medium, which forms yellow bismuth potassium iodide. Based on the reaction, the bismuth content measurement by the ultraviolet-visible spectrophotometry method is established, which is able to accurately measure the bismuth content in the dissolution liquid of the preparation containing the colloidal bismuth pectin.

The present invention provides the method for detecting the dissolution rate of the preparation containing the colloidal bismuth pectin based on characteristics of the colloidal bismuth pectin, wherein the method of the present has a high recovery rate and a good repeatability, in such a manner that dissolution curves and the dissolution rate of the preparation containing the colloidal bismuth pectin are sufficiently detected, so as to better ensure homogeneity, effectiveness and stability. The method of the present invention is able to be used to displace disintegration time limit test for more truly reflecting an intrinsic quality of the preparation, which is quite important for enhancing quality controllability of products and ensuring product quality.

### BRIEF DESCRIPTION OF THE DRAWINGS

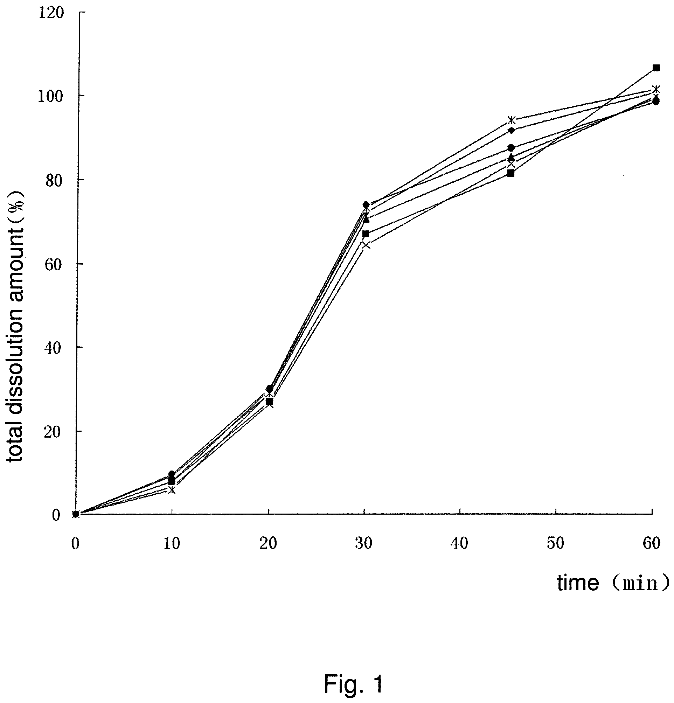

Fig. 1 is dissolution rate curves of colloidal bismuth pectin capsules in water.
Fig. 1 is dissolution rate curves of colloidal bismuth pectin tablets in a phosphate buffer solution.

### DETAILED DESCRIPTION OF THE PREFERRED EMBODIMENT

Embodiment 1: colloidal bismuth pectin dispersible tablet (50mg accounted by bismuth)
   1) Preparation of a chromogen solution: placing 5g ascorbic acid and 25g potassium iodide in a 200ml volumetric flask; adding 100ml of water; shaking to dissolve; adding 25ml of a nitric acid solution of 1mol/L; adding water to dilute; dripping water to meet a scale, so as to obtain the chromogen solution containing 2.5% of the ascorbic acid and 12.5% of potassium iodide.
   2) Preparation of a reference solution of bismuth: placing 275mg accurately weighed bismuth in a 100ml volumetric flask; adding 6.4ml nitric acid to dissolve the bismuth; diluting with water until a scale is reached, so as to obtain a standard bismuth stock solution; accurately weighing 2ml of the standard bismuth stock solution to place in a 100ml volumetric flask; diluting with a 1mol/L nitric acid solution until a scale is reached, so as to obtain a solution containing about 55µg bismuth per 1ml as a standard bismuth solution; accurately weighing 5ml of the standard bismuth solution to place in a 25ml volumetric flask; diluting with the chromogen solution until a scale is reached, so as to obtain the reference solution of the bismuth.
   3) Preparation of a test solution: taking the colloidal bismuth pectin dispersible tablet; according to a second method (paddle apparatus) of dissolution detection in "Pharmacopoeia of the People's Republic of China, 2010 Edition, Volume 2, Appendix XC", using 900ml water as dissolution medium, dissolving for 20min with a rate of 100r/min, and obtaining dissolution liquid; accurately weighing 5ml to place in a 15ml centrifugal tube; accurately adding 5ml of 2mol/L nitric acid solution; shaking for 10 minutes; centrifuging for 10min at 8000r/min; accurately measuring 5ml of supernatant to place in a 25ml volumetric flask; diluting with the chromogen solution until a scale is reached, so as to obtain the test solution.
   4) Preparation of a blank solution: accurately measuring 5ml water to place in a 15ml centrifugal tube; accurately adding 5ml of 2mol/L nitric acid solution; shaking for 5 minutes; accurately measuring 5ml to place in a 25ml volumetric flask; diluting with the chromogen solution until a scale is reached, so as to obtain the blank solution.
   5) Measuring: taking the reference solution and the test solution of the bismuth; adopting the blank solution as a reference; applying ultraviolet-visible spectrophotometry, using a 1cm quartz cuvette, measuring an absorbance at a wavelength of 463nm, and calculating the colloidal bismuth pectin content accounted by the bismuth with an external standard method; comparing with a colloidal bismuth pectin nominal value of the colloidal bismuth pectin dispersible tablet for calculating a dissolution rate.
      According to results, the dissolution rates of six colloidal bismuth pectin dispersible tablets are respectively 96.2%, 92.5%, 94.3%, 95.6%, 91.5%, and 93.3%; an average value is 94%.
Embodiment 2: colloidal bismuth pectin enteric-coated tablet (100mg accounted by bismuth)
   1) Preparation of a chromogen solution: placing 15g citric acid and 50g potassium iodide in a 200ml volumetric flask; adding 100ml of water; shaking to dissolve; adding 25ml of a nitric acid solution of 2mol/L; adding water to dilute; dripping water to meet a scale, so as to obtain the chromogen solution containing 7.5% of the citric acid and 25% of potassium iodide.
   2) Preparation of a reference solution of bismuth: placing 275mg accurately weighed bismuth in a 100ml volumetric flask; adding 6.4ml nitric acid to dissolve the bismuth; diluting with water until a scale is reached, so as to obtain a standard bismuth stock solution; weighing 2ml of the standard bismuth stock solution to place in a 50ml volumetric flask; diluting with a 1.2mol/L nitric acid solution until a scale is reached, so as to obtain a solution containing about 110µg bismuth per 1ml as a standard bismuth solution; accurately weighing 5ml of the standard bismuth solution to place in a 25ml volumetric flask; diluting with the chromogen solution until a scale is reached, so as to obtain the reference solution of the bismuth.
   3) Preparation of a test solution: taking the colloidal bismuth pectin enteric-coated tablet; according to a first method (basket apparatus) of dissolution detection in *"*Pharmacopoeia of the People's Republic of China, 2010 Edition, Volume 2, Appendix XC", using 900ml phosphate buffer solution with a pH of 6.8 as dissolution medium, dissolving for 60min with a rate of 50r/min, and obtaining dissolution liquid; accurately weighing 5ml to place in a centrifugal tube; accurately adding 5ml of 2.4mol/L nitric acid solution; shaking for 5 minutes; centrifuging for 15min at 7000r/min; accurately measuring 5ml of supernatant to place in a 25ml volumetric flask; diluting with the chromogen solution until a scale is reached, so as to obtain the test solution.
   4) Preparation of a blank solution: accurately measuring 5ml water to place in a 15ml centrifugal tube; accurately adding 5ml of 2.4mol/L nitric acid solution; shaking for 5 minutes; accurately measuring 5ml to place in a 25ml volumetric flask; diluting with the chromogen solution until a scale is reached, so as to obtain the blank solution.
   5) Measuring: taking the reference solution and the test solution of the bismuth; adopting the blank solution as a reference; applying ultraviolet-visible spectrophotometry, using a 1cm quartz cuvette, measuring an absorbance at a wavelength of 399nm, and calculating the colloidal bismuth pectin content accounted by the bismuth with an external standard method; comparing with a colloidal bismuth pectin nominal value of the colloidal bismuth pectin enteric-coated tablet for calculating a dissolution rate.

   According to results, the dissolution rates of six colloidal bismuth pectin enteric-coated tablets are respectively 94.2%, 93.2%, 96.3%, 94.8%, 96.5%, and 96.1%; an average value is 95%.
Embodiment 3: colloidal bismuth pectin capsule (50mg accounted by bismuth)
   1) Preparation of a chromogen solution: placing 1g citric acid and 5g potassium iodide in a 200ml volumetric flask; adding 100ml of water; shaking to dissolve; adding 25ml of a nitric acid solution of 10mol/L; adding water to dilute; dripping water to meet a scale, so as to obtain the chromogen solution containing 0.5% of the citric acid and 2.5% of potassium iodide.
   2) Preparation of a reference solution of bismuth: placing 275mg accurately weighed bismuth in a 100ml volumetric flask; adding 6.4ml nitric acid to dissolve the bismuth; diluting with water until a scale is reached, so as to obtain a standard bismuth stock solution; weighing 1ml of the standard bismuth stock solution to place in a 100ml volumetric flask; diluting with a 1.1mol/L hydrochloric acid solution until a scale is reached, so as to obtain a solution containing about 27.5µg bismuth per 1ml as a standard bismuth solution; accurately weighing 1ml of the standard bismuth solution to place in a 25ml volumetric flask; diluting with the chromogen solution until a scale is reached, so as to obtain the reference solution of the bismuth.
   3) Preparation of a test solution: taking the colloidal bismuth pectin capsule; according to a first method (basket apparatus) of dissolution detection in *"*Pharmacopoeia of the People's Republic of China, 2010 Edition, Volume 2, Appendix XC", using 1000ml acetate buffer solution with a pH of 4.0 as dissolution medium, dissolving for 45min with a rate of 75r/min, and obtaining dissolution liquid; accurately weighing 5ml to place in a centrifugal tube; accurately adding 5ml of 2.2mol/L hydrochloric acid solution; shaking for 5 minutes; centrifuging for 5min at 10000r/min; accurately measuring 1ml of supernatant to place in a 25ml volumetric flask; diluting with the chromogen solution until a scale is reached, so as to obtain the test solution.
   4) Preparation of a blank solution: accurately measuring 5ml water to place in a 15ml centrifugal tube; accurately adding 5ml of 2.2mol/L hydrochloric acid solution; shaking for 5 minutes; accurately measuring 1ml to place in a 25ml volumetric flask; diluting with the chromogen solution until a scale is reached, so as to obtain the blank solution.
   5) Measuring: taking the reference solution and the test solution of the bismuth; adopting the blank solution as a reference; applying ultraviolet-visible spectrophotometry, using a 1cm quartz cuvette, measuring an absorbance at a wavelength of 399nm, and calculating the colloidal bismuth pectin content accounted by the bismuth with an external standard method; comparing with a colloidal bismuth pectin nominal value of the colloidal bismuth pectin capsule for calculating a dissolution rate.

   According to results, the dissolution rates of six colloidal bismuth pectin capsules are respectively 96.5%, 98.7%, 95.4%, 97.3%, 96.1%, and 94.7%; an average value is 96%.
Embodiment 4: colloidal bismuth pectin colon-enteric-coated capsule (50mg accounted by bismuth)
   1) Preparation of a chromogen solution: placing 20g ascorbic acid and 20g potassium iodide in a 200ml volumetric flask; adding 100ml of water; shaking to dissolve; adding 50ml of a nitric acid solution of 2mol/L; adding water to dilute; dripping water to meet a scale, so as to obtain the chromogen solution containing 10% of the ascorbic acid and 10% of potassium iodide.
   2) Preparation of a reference solution of bismuth: placing 275mg accurately weighed bismuth in a 100ml volumetric flask; adding 6.4ml nitric acid to dissolve the bismuth; diluting with water until a scale is reached, so as to obtain a standard bismuth stock solution; weighing 1ml of the standard bismuth stock solution to place in a 25ml volumetric flask; diluting with a 0.6mol/L sulfuric acid solution until a scale is reached, so as to obtain a solution containing about 110µg bismuth per 1ml as a standard bismuth solution; accurately weighing 10ml of the standard bismuth solution to place in a 25ml volumetric flask; diluting with the chromogen solution until a scale is reached, so as to obtain the reference solution of the bismuth.
   3) Preparation of a test solution: taking the colloidal bismuth pectin colon-enteric-coated capsule; according to a third method (cup apparatus) of dissolution detection in "Pharmacopoeia of the People's Republic of China, 2010 Edition, Volume 2, Appendix XC", using 200ml sodium borate buffer solution with a pH of 8.5 as dissolution medium, dissolving for 90min with a rate of 30r/min, and obtaining dissolution liquid; accurately weighing 5ml to place in a centrifugal tube; accurately adding 5ml of 1.2mol/L sulfuric acid solution; shaking for 5 minutes; centrifuging for 15min at 8000r/min; accurately measuring 10ml of supernatant to place in a 25ml volumetric flask; diluting with the chromogen solution until a scale is reached, so as to obtain the test solution.
   4) Preparation of a blank solution: accurately measuring 5ml water to place in a 15ml centrifugal tube; accurately adding 5ml of 1.2mol/L sulfuric acid solution; shaking for 5 minutes; accurately measuring 10ml to place in a 25ml volumetric flask; diluting with the chromogen solution until a scale is reached, so as to obtain the blank solution.
   5) Measuring: taking the reference solution and the test solution of the bismuth; adopting the blank solution as a reference; applying ultraviolet-visible spectrophotometry, using a 1cm quartz cuvette, measuring an absorbance at a wavelength of 433nm, and calculating the colloidal bismuth pectin content accounted by the bismuth with an external standard method; comparing with a colloidal bismuth pectin nominal value of the colloidal bismuth pectin colon-enteric-coated capsule for calculating a dissolution rate.

   According to results, the dissolution rates of six colloidal bismuth pectin colon-enteric-coated capsules are respectively 98.2%, 97.3%, 96.9%, 99.6%, 97.8%, and 99.2%; an average value is 98%.
Embodiment 5: colloidal bismuth pectin powder (50mg accounted by bismuth)
   1) Preparation of a chromogen solution: placing lOg ascorbic acid and 30g potassium iodide in a 200ml volumetric flask; adding 100ml of water; shaking to dissolve; adding 25ml of a nitric acid solution of 5mol/L; adding water to dilute; dripping water to meet a scale, so as to obtain the chromogen solution containing 5% of the ascorbic acid and 15% of potassium iodide.
   2) Preparation of a reference solution of bismuth: placing 250mg accurately weighed bismuth in a 50ml volumetric flask; adding 6.4ml nitric acid to dissolve the bismuth; diluting with water until a scale is reached, so as to obtain a standard bismuth stock solution; weighing 1ml of the standard bismuth stock solution to place in a 50ml volumetric flask; diluting with a 1.1mol/L nitric acid solution until a scale is reached, so as to obtain a solution containing about 100µg bismuth per 1ml as a standard bismuth solution; accurately weighing 5ml of the standard bismuth solution to place in a 25ml volumetric flask; diluting with the chromogen solution until a scale is reached, so as to obtain the reference solution of the bismuth.
   3) Preparation of a test solution: taking the colloidal bismuth pectin powder; according to a second method (paddle apparatus) of dissolution detection in "Pharmacopoeia of the People's Republic of China, 2010 Edition, Volume 2, Appendix XC", using 500ml phthalate buffer solution with a pH of 5.8 as dissolution medium, dissolving for 120min with a rate of 30r/min, and obtaining dissolution liquid; accurately weighing 5ml to place in a centrifugal tube; accurately adding 5ml of 2.2mol/L nitric acid solution; shaking for 5 minutes; centrifuging for 10min at 10000r/min; accurately measuring 5ml of supernatant to place in a 25ml volumetric flask; diluting with the chromogen solution until a scale is reached, so as to obtain the test solution.
   4) Preparation of a blank solution: accurately measuring 5ml water to place in a 15ml centrifugal tube; accurately adding 5ml of 2.2mol/L nitric acid solution; shaking for 5 minutes; accurately measuring 5ml to place in a 25ml volumetric flask; diluting with the chromogen solution until a scale is reached, so as to obtain the blank solution.
   5) Measuring: taking the reference solution and the test solution of the bismuth; adopting the blank solution as a reference; applying ultraviolet-visible spectrophotometry, using a 1cm quartz cuvette, measuring an absorbance at wavelengths of 433nm and 463nm, and calculating the colloidal bismuth pectin content accounted by the bismuth with an external standard method according to an absorbance difference at the two wavelengths; comparing with a colloidal bismuth pectin nominal value of the colloidal bismuth pectin powder for calculating a dissolution rate.

   According to results, the dissolution rates of six pockets of the colloidal bismuth pectin powder are respectively 95.1%, 99.3%, 97.9%, , 96.2%, 95.6%, and 98.3%; an average value is 97%.
Embodiment 6: colloidal bismuth pectin granule (150mg accounted by bismuth)
   1) Preparation of a chromogen solution: placing 5g ascorbic acid and 40g potassium iodide in a 200ml volumetric flask; adding 100ml of water; shaking to dissolve; adding 25ml of a nitric acid solution of 2mol/L; adding water to dilute; dripping water to meet a scale, so as to obtain the chromogen solution containing 2.5% of the ascorbic acid and 20% of potassium iodide.
   2) Preparation of a reference solution of bismuth: placing 500mg accurately weighed bismuth in a 100ml volumetric flask; adding 6.4ml nitric acid to dissolve the bismuth; diluting with water until a scale is reached, so as to obtain a standard bismuth stock solution; weighing 1ml of the standard bismuth stock solution to place in a 50ml volumetric flask; diluting with a 0.9mol/L nitric acid solution until a scale is reached, so as to obtain a solution containing about 100µg bismuth per 1ml as a standard bismuth solution; accurately weighing 2ml of the standard bismuth solution to place in a 25ml volumetric flask; diluting with the chromogen solution until a scale is reached, so as to obtain the reference solution of the bismuth.
   3) Preparation of a test solution: taking the colloidal bismuth pectin granule; according to a second method (paddle apparatus) of dissolution detection in "Pharmacopoeia of the People's Republic of China, 2010 Edition, Volume 2, Appendix XC", using 750ml 0.01mol/L hydrochloric solution as dissolution medium, dissolving for 30min with a rate of 50r/min, and obtaining dissolution liquid; accurately weighing 5ml to place in a centrifugal tube; accurately adding 5ml of 1.8mol/L nitric acid solution; shaking for 5 minutes; centrifuging for 10min at 7000r/min; accurately measuring 2ml of supernatant to place in a 25ml volumetric flask; diluting with the chromogen solution until a scale is reached, so as to obtain the test solution.
   4) Preparation of a blank solution: accurately measuring 5ml water to place in a 15ml centrifugal tube; accurately adding 5ml of 1.8mol/L nitric acid solution; shaking for 5 minutes; accurately measuring 2ml to place in a 25ml volumetric flask; diluting with the chromogen solution until a scale is reached, so as to obtain the blank solution.
   5) Measuring: taking the reference solution and the test solution of the bismuth; adopting the blank solution as a reference; applying ultraviolet-visible spectrophotometry, using a 1cm quartz cuvette, measuring an absorbance at wavelengths of 399nm and 463nm, and calculating the colloidal bismuth pectin content accounted by the bismuth with an external standard method according to an absorbance difference at the two wavelengths; comparing with a colloidal bismuth pectin nominal value of the colloidal bismuth pectin granule for calculating a dissolution rate.

   According to results, the dissolution rates of six colloidal bismuth pectin granules are respectively 93.2%, 94.0%, 92.9%, 96.7%, 94.7%, and 90.6%; an average value is 94%.
Embodiment 7: colloidal bismuth pectin dry suspension (150mg accounted by bismuth)
   1) Preparation of a chromogen solution: placing 8g ascorbic acid and 50g potassium iodide in a 200ml volumetric flask; adding 100ml of water; shaking to dissolve; adding water to dilute; dripping water to meet a scale, so as to obtain the chromogen solution containing 4% of the ascorbic acid and 25% of potassium iodide.
   2) Preparation of a reference solution of bismuth: placing 250mg accurately weighed bismuth in a 100ml volumetric flask; adding 6.4ml nitric acid to dissolve the bismuth; diluting with water until a scale is reached, so as to obtain a standard bismuth stock solution; weighing 5ml of the standard bismuth stock solution to place in a 25ml volumetric flask; diluting with a 0.8mol/L nitric acid solution until a scale is reached, so as to obtain a solution containing about 500µg bismuth per 1ml as a standard bismuth solution; accurately weighing 2ml of the standard bismuth solution to place in a 25ml volumetric flask; diluting with the chromogen solution until a scale is reached, so as to obtain the reference solution of the bismuth.
   3) Preparation of a test solution: taking the colloidal bismuth pectin dry suspension; according to a third method (cup apparatus) of dissolution detection in "Pharmacopoeia of the People's Republic of China, 2010 Edition, Volume 2, Appendix XC", using 150ml 0.1mol/L hydrochloric acid solution as dissolution medium, dissolving for 60min with a rate of 45r/min, and obtaining dissolution liquid; accurately weighing 5ml to place in a centrifugal tube; accurately adding 5ml of 1.6mol/L nitric acid solution; shaking for 5 minutes; centrifuging for 15min at 7000r/min; accurately measuring 2ml of supernatant to place in a 25ml volumetric flask; diluting with the chromogen solution until a scale is reached, so as to obtain the test solution.
   4) Preparation of a blank solution: accurately measuring 5ml water to place in a 15ml centrifugal tube; accurately adding 5ml of 1.6mol/L nitric acid solution; shaking for 5 minutes; accurately measuring 2ml to place in a 25ml volumetric flask; diluting with the chromogen solution until a scale is reached, so as to obtain the blank solution.
   5) Measuring: taking the reference solution and the test solution of the bismuth; adopting the blank solution as a reference; applying ultraviolet-visible spectrophotometry, using a 1cm quartz cuvette, measuring an absorbance at a wavelength of 433nm, and calculating the colloidal bismuth pectin content accounted by the bismuth with an external standard method; comparing with a colloidal bismuth pectin nominal value of the colloidal bismuth pectin dry suspension for calculating a dissolution rate.

   According to results, the dissolution rates of six pockets of colloidal bismuth pectin dry suspension are respectively 95.2%, 93.3%, 91.6%, 92.9%, 93.0%, and 90.1%; an average value is 93%.
Embodiment 8: compound colloidal bismuth pectin capsule (comprising colloidal bismuth pectin, metronidazole and tetracycline hydrochloride, wherein 35mg accounted by bismuth for each capsule)
   1) Preparation of a chromogen solution: placing 15g citric acid and 30g potassium iodide in a 200ml volumetric flask; adding 100ml of water; shaking to dissolve; adding 25ml of 1mol/L nitric acid solution; adding water to dilute; dripping water to meet a scale, so as to obtain the chromogen solution containing 7.5% of the citric acid and 15% of potassium iodide.
   2) Preparation of a reference solution of bismuth: placing 250mg accurately weighed bismuth in a 100ml volumetric flask; adding 6.4ml nitric acid to dissolve the bismuth; diluting with water until a scale is reached, so as to obtain a standard bismuth stock solution; weighing 3ml of the standard bismuth stock solution to place in a 100ml volumetric flask; diluting with a 1.2mol/L hydrochloric acid solution until a scale is reached, so as to obtain a solution containing about 75µg bismuth per 1ml as a standard bismuth solution; accurately weighing 5ml of the standard bismuth solution to place in a 25ml volumetric flask; diluting with the chromogen solution until a scale is reached, so as to obtain the reference solution of the bismuth.
   3) Preparation of a test solution: taking the compound colloidal bismuth pectin capsule; according to a third method (cup apparatus) of dissolution detection in "Pharmacopoeia of the People's Republic of China, 2010 Edition, Volume 2, Appendix XC", using 250ml 0.01mol/L hydrochloric acid solution as dissolution medium, dissolving for 5min with a rate of 150r/min, and obtaining dissolution liquid; accurately weighing 5ml to place in a centrifugal tube; accurately adding 5ml of 2.4mol/L hydrochloric acid solution; shaking for 10 minutes; centrifuging for 12min at 7000r/min; accurately measuring 5ml of supernatant to place in a 25ml volumetric flask; diluting with the chromogen solution until a scale is reached, so as to obtain the test solution.
   4) Preparation of a blank solution: accurately measuring 5ml water to place in a 15ml centrifugal tube; accurately adding 5ml of 2.4mol/L hydrochloric acid solution; shaking for 10 minutes; accurately measuring 5ml to place in a 25ml volumetric flask; diluting with the chromogen solution until a scale is reached, so as to obtain the blank solution.
   5) Measuring: taking the reference solution and the test solution of the bismuth; adopting the blank solution as a reference; applying ultraviolet-visible spectrophotometry, using a 1cm quartz cuvette, measuring an absorbance at a wavelength of 399nm, and calculating the colloidal bismuth pectin content accounted by the bismuth with an external standard method; comparing with a colloidal bismuth pectin nominal value of the compound colloidal bismuth pectin capsule for calculating a dissolution rate.

   According to results, the dissolution rates of six compound colloidal bismuth pectin capsules are respectively 92.3%, 91.6%, 90.9%, 92.1%, 89.6%, and 94.1%; an average value is 92%.
Embodiment 9: detection of dissolution curve of colloidal bismuth pectin capsule (100mg accounted by bismuth)
   1) Preparation of a chromogen solution: placing 5g ascorbic acid and 25g potassium iodide in a 200ml volumetric flask; adding 100ml of water; shaking to dissolve; adding 25ml of a nitric acid solution of 1mol/L; adding water to dilute; dripping water to meet a scale, so as to obtain the chromogen solution containing 2.5% of the ascorbic acid and 12.5% of potassium iodide.
   2) Preparation of a reference solution of bismuth: placing 275mg accurately weighed bismuth in a 100ml volumetric flask; adding 6.4ml nitric acid to dissolve the bismuth; diluting with water until a scale is reached, so as to obtain a standard bismuth stock solution; weighing 2ml of the standard bismuth stock solution to place in a 100ml volumetric flask; diluting with a 1mol/L nitric acid solution until a scale is reached, so as to obtain a solution containing about 55µg bismuth per 1ml as a standard bismuth solution; accurately weighing 5ml of the standard bismuth solution to place in a 25ml volumetric flask; diluting with the chromogen solution until a scale is reached, so as to obtain the reference solution of the bismuth.
   3) Preparation of a test solution: taking the colloidal bismuth pectin capsule; according to a second method (paddle apparatus) of dissolution detection in "Pharmacopoeia of the People's Republic of China, 2010 Edition, Volume 2, Appendix XC", using 900ml water as dissolution medium, dissolving for 60min with a rate of 75r/min, and obtaining 10ml dissolution liquid respectively at 10min, 20min, 30min, 45min and 60min; accurately weighing 5ml the dissolution liquid to place in a centrifugal tube; accurately adding 5ml of 2mol/L nitric acid solution; shaking and then centrifuging for 10min at 8000r/min; accurately measuring 5ml of supernatant to place in a 25ml volumetric flask; diluting with the chromogen solution until a scale is reached, so as to obtain the test solution.
   4) Preparation of a blank solution: accurately measuring 5ml water to place in a 15ml centrifugal tube; accurately adding 5ml of 2mol/L nitric acid solution; shaking for 10 minutes; accurately measuring 5ml to place in a 25ml volumetric flask; diluting with the chromogen solution until a scale is reached, so as to obtain the blank solution.
   5) Measuring: taking the reference solution and the test solution of the bismuth; adopting the blank solution as a reference; applying ultraviolet-visible spectrophotometry, using a 1cm quartz cuvette, measuring an absorbance at a wavelength of 463nm, and calculating the colloidal bismuth pectin content accounted by the bismuth with an external standard method; comparing with a colloidal bismuth pectin nominal value of the colloidal bismuth pectin capsule for calculating dissolution rates at different time points for drawing the dissolution curve.

   According to results, average dissolution rates of six colloidal bismuth pectin capsules at 10min, 20min, 30min, 45min and 60min are respectively 7.8%, 28.5%, 70.3%, 87.4%, and 101.3%; the dissolution curve is shown as Fig. 1.
Embodiment 10: detection of dissolution curve of colloidal bismuth pectin tablet (50mg accounted by bismuth)
   1) Preparation of a chromogen solution: placing 10g citric acid and 25g potassium iodide in a 200ml volumetric flask; adding 100ml of water; shaking to dissolve; adding 25ml of a nitric acid solution of 4mol/L; adding water to dilute; dripping water to meet a scale, so as to obtain the chromogen solution containing 5% of the citric acid and 12.5% of potassium iodide.
   2) Preparation of a reference solution of bismuth: placing 275mg accurately weighed bismuth in a 100ml volumetric flask; adding 6.4ml nitric acid to dissolve the bismuth; diluting with water until a scale is reached, so as to obtain a standard bismuth stock solution; weighing 1ml of the standard bismuth stock solution to place in a 100ml volumetric flask; diluting with a 1.2mol/L nitric acid solution until a scale is reached, so as to obtain a solution containing about 27.5µg bismuth per 1ml as a standard bismuth solution; accurately weighing 5ml of the standard bismuth solution to place in a 25ml volumetric flask; diluting with the chromogen solution until a scale is reached, so as to obtain the reference solution of the bismuth.
   3) Preparation of a test solution: taking the colloidal bismuth pectin tablet; according to a first method (basket apparatus) of dissolution detection in "Pharmacopoeia of the People's Republic of China, 2010 Edition, Volume 2, Appendix XC", using 900ml phosphate buffer solution with a pH of 6.8 as dissolution medium, dissolving for 60min with a rate of 100r/min, and obtaining 10ml dissolution liquid respectively at 10min, 20min, 30min, 45min and 60min; accurately weighing 5ml the dissolution liquid to place in a centrifugal tube; accurately adding 5ml of 2.4mol/L nitric acid solution; shaking and then centrifuging for 5min at 10000r/min; accurately measuring 5ml of supernatant to place in a 25ml volumetric flask; diluting with the chromogen solution until a scale is reached, so as to obtain the test solution.
   4) Preparation of a blank solution: accurately measuring 5ml water to place in a 15ml centrifugal tube; accurately adding 5ml of 2.4mol/L nitric acid solution; shaking for 5 minutes; accurately measuring 5ml to place in a 25ml volumetric flask; diluting with the chromogen solution until a scale is reached, so as to obtain the blank solution.
   5) Measuring: taking the reference solution and the test solution of the bismuth; adopting the blank solution as a reference; applying ultraviolet-visible spectrophotometry, using a 1cm quartz cuvette, measuring an absorbance at a wavelength of 463nm, and calculating the colloidal bismuth pectin content accounted by the bismuth with an external standard method; comparing with a colloidal bismuth pectin nominal value of the colloidal bismuth pectin tablet for calculating dissolution rates at different time points for drawing the dissolution curve.

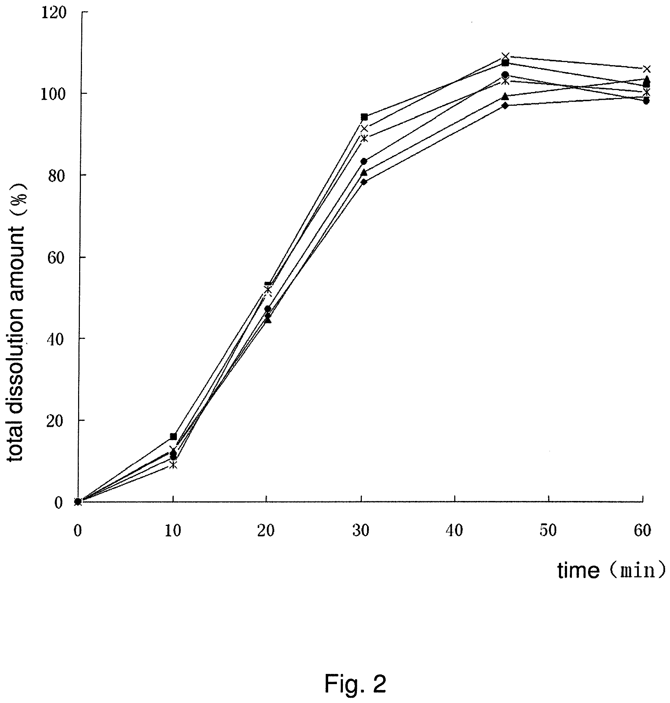

   According to results, average dissolution rates of six colloidal bismuth pectin capsules at 10min, 20min, 30min, 45min and 60min are respectively 12.3%, 48.9%, 86.2%, 103.6%, 101.7%; the dissolution curve is shown as Fig. 2.

## Claims

1. A method for detecting a dissolution rate of a preparation containing colloidal bismuth pectin, comprising steps of: according to "*Pharmacopoeia of the People's Republic of China*, 2010 Edition, Volume 2, Appendix XC", dispersing the preparation containing the colloidal bismuth pectin into dissolution medium, dissolving for 3-120min with a centrifuging rate of 30-150r/min, and directly obtaining dissolution liquid; adding a protonic acid dissociation agent into the dissolution liquid until a hydrogen ion concentration reaches 0.8-1.2mol/L; centrifuging after completely dissociation; separating a supernatant; coloring the supernatant by adding a chromogen solution of citric acid or ascorbic acid and potassium iodide to obtain a test solution; testing an absorbance of the test solution at a wavelength of 380-470nm; comparing the absorbance of the test solution with an absorbance of a reference solution with a known bismuth concentration under same conditions; calculating a dissolution amount, which is accounted by bismuth, of the colloidal bismuth pectin; wherein the dissolution medium is water, a buffer solution with a pH of 1.0-10.0, or an acid solution.

2. The method, as recited in claim 1, wherein the preparation containing the colloidal bismuth pectin is a single preparation or a compound preparation, which comprises tablets, dispersible tablets, enteric-coated tablets, colon-enteric-coated tablets, capsules, soft capsules, enteric-coated capsules, colon-enteric-coated capsules, granules, dripping pills, microcapsules and dry suspension.

3. The method, as recited in claim 1, wherein the buffer solution is an acetate buffer solution, a phthalate buffer solution, a phosphate buffer solution or a borate buffer solution, with a pH of 1.0-10.0.

4. The method, as recited in claim 1, wherein the acid solution is a 0.005-0.1mol/L hydrochloric acid solution.

5. The method, as recited in claim 1, wherein the dissolution detection method is a first method being the basket apparatus method, a second method being the paddle apparatus method or a third method being the cup apparatus method in the "*Pharmacopoeia of the People's Republic of China*, Volume 2, Appendix XC".

6. The method, as recited in claim 1, wherein the protonic acid dissociation agent is nitric acid, hydrochloric acid or sulfuric acid.

7. The method, as recited in claim 1, wherein the chromogen solution is a water solution or a 0.2-2mol/L nitric acid solution of the potassium iodide, with the citric acid or the ascorbic acid.

8. The method, as recited in claim 7, wherein the chromogen solution comprises the citric acid or the ascorbic acid of 0.5wt%-10wt%, and the potassium iodide of 2.5wt %-25wt%.

9. The method, as recited in claim 1, wherein the reference solution of the bismuth is prepared by dissolving metal bismuth in nitric acid before being diluted with water and adding the chromogen solution.

10. The method, as recited in claim 1, wherein a bismuth content per 1ml of the test solution or 1ml of the reference solution of the bismuth is 0.1-50µg.

11. The method, as recited in claim 1, wherein the dissolution liquid dissociated with the protonic acid is centrifuged for 5-15min with a centrifuging rate of 7000-10000r/min.

12. The method, as recited in claim 1, wherein a single-wavelength method is adopted for measurement with any wavelength of 399nm, 433nm and 463nm; or a double-wavelength method is adopted for measurement with any two wavelengths of 399nm, 433nm and 463nm.

## Patentansprüche

1. Verfahren zur Ermittlung der Lösungsgeschwindigkeit eines Präparats, das kolloidales Bismutpektin enthält, folgende Schritte aufweisend: gemäß "Pharmacopoeia of the People's Republic of China, 2010 Edition, Volume 2, Appendix XC" (Arzneibuch der Volksrepublik China, Ausgabe 2010, Band 2, Anhang XC), Dispergieren des Präparats, das das kolloidale Bismutpektin enthält, in ein Lösungsmedium, 3 bis 120 min Lösen mit einer Zentrifugiergeschwindigkeit von 30 bis 150 U/min und direkt Erhalten einer Lösungsflüssigkeit; Zugeben eines Protonendonator-Dissoziationsmittels in die Lösungsflüssigkeit bis die Wasserstoffionen-Konzentration 0,8 bis 1,2 mol/l erreicht; Zentrifugieren nach vollständiger Dissoziation; Abtrennen eines Überstands; Färben des Überstands durch Zugeben einer Chromogen-Lösung von Zitronensäure oder Ascorbinsäure und Kaliumiodid, um eine Testlösung zu erhalten; Testen der Extinktion der Testlösung bei einer Wellenlänge von 380 bis 470 nm; Vergleichen der Extinktion der Testlösung mit der Extinktion einer Referenzlösung mit einer bekannten Bismut-Konzentration unter den selben Bedingungen; Berechnen einer Lösungsmenge des kolloidalen Bismutpektins, die auf Bismut entfällt; wobei das Lösungsmedium Wasser, eine Pufferlösung mit einem pH von 1,0 bis 10,0 oder eine Säurelösung ist.

2. Verfahren wie in Anspruch 1 angegeben, wobei das Präparat, das das kolloidale Bismutpektin enthält, ein Einzelpräparat oder ein Verbundpräparat ist, das Tabletten, dispergierbare Tabletten, magensaftresistent-beschichtete Tabletten, colon-magensaftresistent-beschichtete Tabletten, Kapseln, Weichkapseln, magensaftresistent-beschichtete Kapseln, colon-magensaftresistent-beschichtete Kapseln, Granulat, Tropfpillen, Mikrokapseln und Trockensaft aufweist.

3. Verfahren wie in Anspruch 1 angegeben, wobei die Pufferlösung eine Acetat-Pufferlösung, eine Phthalat-Pufferlösung, eine Phosphat-Pufferlösung oder eine Borat-Pufferlösung mit einem pH von 1,0 bis 10,0 ist.

4. Verfahren wie in Anspruch 1 angegeben, wobei die Säurelösung eine 0,005 bis 0,1 mol/l Salzsäurelösung ist.

5. Verfahren wie in Anspruch 1 angegeben, wobei das Lösungs-Ermittlungsverfahren ein erstes Verfahren, das das Rotationskorbvorrichtungs-Verfahren ist, ein zweites Verfahren, das das Paddelvorrichtungs-Verfahren ist, oder ein drittes Verfahren, das das Bechervorrichtungs-Verfahren ist, in dem "Pharmacopoeia of the People's Republic of China, Volume 2, Appendix XC" (Arzneibuch der Volksrepublik China, Band 2, Anhang XC) ist.

6. Verfahren wie in Anspruch 1 angegeben, wobei das Protonendonator-Dissoziationsmittel Salpetersäure, Salzsäure oder Schwefelsäure ist.

7. Verfahren wie in Anspruch 1 angegeben, wobei die Chromogen-Lösung eine Wasserlösung oder eine 0,2 bis 2 mol/l Salpetersäure-Lösung des Kaliumiodids mit der Zitronensäure oder der Ascorbinsäure ist.

8. Verfahren wie in Anspruch 7 angegeben, wobei die Chromogen-Lösung 0,5 Gew.% bis 10 Gew.% Zitronensäure oder Ascorbinsäure und 2,5 Gew.% bis 25 Gew.% Kaliumiodid aufweist.

9. Verfahren wie in Anspruch 1 angegeben, wobei die Referenzlösung des Bismuts hergestellt wird durch Lösen von Bismutmetall in Salpetersäure, bevor sie mit Wasser verdünnt wird, und Zugeben der Chromogen-Lösung.

10. Verfahren wie in Anspruch 1 angegeben, wobei der Bismut-Gehalt pro 1 ml der Testlösung oder 1 ml der Referenzlösung des Bismuts 0,1 bis 50 µg ist.

11. Verfahren wie in Anspruch 1 angegeben, wobei die mit dem Protonendonator dissoziierte Lösungsflüssigkeit 5 bis 15 Minuten lang mit einer Zentrifugiergeschwindigkeit von 7.000 bis 10.000 U/min zentrifugiert wird.

12. Verfahren wie in Anspruch 1 angegeben, wobei zur Messung ein Verfahren mit einer einzigen Wellenlänge mit irgendeiner der Wellenlängen 399 nm, 433 nm und 463 nm gewählt wird; oder ein Verfahren mit zwei Wellenlängen zur Messung mit beliebigen zwei Wellenlängen der Wellenlängen 399 nm, 433 nm und 463 nm gewählt wird.

## Revendications

1. Procédé pour détecter un taux de dissolution d'une préparation qui contient de la pectine de bismuth colloïdal, comprenant les étapes constituées par : conformément à "*Pharmacopoeia of the People's Republic of China*, Edition 2010, Volume 2, Annexe XC", la dispersion de la préparation qui contient la pectine de bismuth colloïdal dans un milieu de dissolution, la dissolution pendant 3 à 120 minutes selon une vitesse de centrifugation de 30 à 150 tours/minute et l'obtention de façon directe d'un liquide de dissolution ; l'ajout d'un agent de dissociation d'acide protonique dans le liquide de dissolution jusqu'à ce qu'une concentration en ions d'hydrogène atteigne 0,8 à 1,2 mol/L ; la centrifugation après dissociation complète ; la séparation d'un surnageant ; la coloration du surnageant en ajoutant une solution chromogène d'acide citrique ou d'acide ascorbique et d'iodure de potassium pour obtenir une solution de test ; le test d'une absorbance de la solution de test à une longueur d'onde de 380 à 470 nm ; la comparaison de l'absorbance de la solution de test avec une absorbance d'une solution de référence qui présente une concentration en bismuth connue sous les mêmes conditions ; le calcul d'une valeur de dissolution, qui est attribuable au bismuth, de la pectine de bismuth colloïdal ; dans lequel le milieu de dissolution est de l'eau, une solution tampon qui présente un pH de 1,0 à 10,0 ou une solution d'acide.

2. Procédé tel que revendiqué selon la revendication 1, dans lequel la préparation qui contient la pectine de bismuth colloïdal est une préparation simple ou une préparation composite, laquelle comprend des comprimés, des comprimés dispersibles, des comprimés à enrobage entérique, des comprimés à enrobage entérique pour le côlon, des gélules, des gélules à enveloppe molle, des gélules à enrobage entérique, des gélules à enrobage entérique pour le côlon, des granules, des cachets pour instillation, des microgélules et une suspension sèche.

3. Procédé tel que revendiqué selon la revendication 1, dans lequel la solution tampon est une solution tampon constituée par de l'acétate, une solution tampon constituée par du phtalate, une solution tampon constituée par du phosphate ou une solution tampon constituée par du borate, laquelle présente un pH de 1,0 à 10,0.

4. Procédé tel que revendiqué selon la revendication 1, dans lequel la solution d'acide est une solution d'acide chlorhydrique à 0,005 à 0,1 mol/L.

5. Procédé tel que revendiqué selon la revendication 1, dans lequel le procédé de détection de dissolution est un premier procédé qui est le procédé par appareil à panier, un deuxième procédé qui est le procédé par appareil à ailettes ou un troisième procédé qui est le procédé par appareil à coupelle selon "*Pharmacopoeia of the People's Republic of China*, Volume 2, Annexe XC".

6. Procédé tel que revendiqué selon la revendication 1, dans lequel l'agent de dissociation d'acide protonique est l'acide nitrique, l'acide chlorhydrique ou l'acide sulfurique.

7. Procédé tel que revendiqué selon la revendication 1, dans lequel la solution chromogène est une solution aqueuse ou une solution d'acide nitrique à 0,2 à 2 mol/L du iodure de potassium, avec l'acide citrique ou l'acide ascorbique.

8. Procédé tel que revendiqué selon la revendication 7, dans lequel la solution chromogène comprend de l'acide citrique ou de l'acide ascorbique à raison de 0,5 % en poids à 10 % en poids, et du iodure de potassium à raison de 2,5 % en poids à 25 % en poids.

9. Procédé tel que revendiqué selon la revendication 1, dans lequel la solution de référence du bismuth est préparée en dissolvant du bismuth sous forme de métal dans de l'acide nitrique avant dilution à l'aide d'eau et ajout de la solution chromogène.

10. Procédé tel que revendiqué selon la revendication 1, dans lequel une teneur en bismuth pour 1 ml de la solution de test ou pour 1 ml de la solution de référence du bismuth est comprise entre 0,1 µg et 50 µg.

11. Procédé tel que revendiqué selon la revendication 1, dans lequel le liquide de dissolution qui est dissocié avec l'acide protonique est centrifugé pendant 5 à 15 minutes à une vitesse de centrifugation de 7 000 à 10 000 tours/minute.

12. Procédé tel que revendiqué selon la revendication 1, dans lequel un procédé à une seule longueur d'onde est adopté pour la mesure avec une longueur d'onde quelconque parmi 399 nm, 433 nm et 463 nm ; ou un procédé à deux longueurs d'onde est adopté pour la mesure avec deux longueurs d'onde quelconques parmi 399 nm, 433 nm et 463 nm.
